# EUROPEAN PATENT SPECIFICATION

(11) **EP 1 917 927 B1**
(45) Date of publication and mention of the grant of the patent: **22.12.2010**
(21) Application number: 07010896.4
(22) Date of filing: 01.06.2007
(51) Int. Cl.: A61B 18/12

(54) **High-frequency operation apparatus for controlling high-frequency output based on change with time of an electrical parameter**
Hochfrequenzoperationsvorrichtung zum Steuern der Hochfrequenzausgangsleistung auf der Basis der zeitlichen Änderung der elektrischen Parameter
Appareil de fonctionnement à hautes fréquences de contrôle de sortie à hautes fréquences basé sur une modification du temps des paramètres électriques

(30) Priority: 31.10.2006 US 555092
(43) Date of publication of application: 07.05.2008
(73) Proprietor: OLYMPUS MEDICAL SYSTEMS CORP., Shibuya-ku Tokyo 151-0072 (JP)
(72) Inventor: Irisawa, Takashi, Hachioji-shi Tokyo 192-8512 (JP); Mihori, Takashi, Hachioji-shi Tokyo 192-8512 (JP); Tanaka, Kazue, Hachioji-shi Tokyo 192-8512 (JP)
(74) Representative: von Hellfeld, Axel

(56) References cited:
- EP-A- 1 598 025
- WO-A-00/53133
- WO-A-03/005918
- WO-A-2007/067522
- US-A- 5 167 658
- US-A1- 2005 101 948

## Description

This invention relates to high-frequency operation apparatus for controlling a high-frequency output based on the change with time of a parameter.

Document US 2005/0101948 A1 concerns an electronic operation apparatus having a high-frequency generation circuit, a current sensor, a voltage sensor and a control circuit according to the preamble of claims 1. High-frequency energy generated by the high-frequency generation circuit is applied to living tissue. For controlling the apparatus, an impedance provided by the living tissue is calculated. If the calculated impedance exceeds a predetermined value, the control circuit discontinues output of high-frequency energy.

Techniques for treating a blood vessel with high-frequency energy in a surgical operation are known. For instance, there are devices for supplying a high-frequency current in a condition where a blood vessel is held by appropriate holding power and sealing off the blood vessel by means of the thermal energy generated at that time. Generally, it is known that, when a tissue is modified, the water in the tissue is lost by dehydration and the impedance that is the electric resistance rises. On the bases of this fact, the above-described device for sealing off a blood vessel controls a high-frequency output by means of the detected impedance.

While the output of a power source that is used in a blood vessel sealing treatment is often on/off-controlled by means of a very low frequency, the on/off timings are controlled according to the detected impedance.

From the above-described facts, it is believed to be rational to control a high-frequency output according to the impedance that indicates the condition of a tissue.

Some or all of the above problems are overcome by a high-frequency operation apparatus having the features of claim 1.

According to a first aspect of the present invention, there is provided a high-frequency operation apparatus comprising: a high-frequency energy generating section which generates and outputs high-frequency energy to be used for treating an object tissue; a parameter monitoring section which monitors the change with time of an electrical parameter indicting the condition of the object tissue when the high-frequency energy output from the high-frequency energy generating section is supplied to the object tissue; and a control section which controls the output of high-frequency energy according to the outcome of the monitoring by the parameter monitoring section.

According to a second aspect of the present invention, in a high-frequency operation apparatus in the first aspect of the invention, the parameter monitoring section monitors the change with time of the impedance that indicates the condition of the object tissue.

According to a third aspect of the present invention, in a high-frequency operation apparatus in the third aspect of the invention, the parameter monitoring section measures the impedance at predetermined time intervals during the treatment, monitors if the impedance exceeds the first threshold value or not and, when the number of times of exceeding the first threshold value gets to a predetermined number of times, the control section stops the output of high-frequency energy.

According to a fourth aspect of the present invention, in a high-frequency operation apparatus in the third aspect of the invention, the parameter monitoring section resets the count value obtained by the past measurement but continues the measurement of the impedance when the impedance falls below the first threshold value during the measurement of the impedance.

According to a fifth aspect of the present invention, in a high-frequency operation apparatus in the third aspect of the invention, the parameter monitoring section determines if the impedance exceeds the second threshold value that is different from the first threshold value or not after the number of times by which the impedance exceeds the first threshold value gets to a predetermined number of times and the control section stops outputting the high-frequency energy when the impedance exceeds the second threshold value.

According to a sixth aspect of the present invention, in a high-frequency operation apparatus in the first aspect of the invention, the parameter monitoring section monitors the amount of electric power of the high-frequency energy being applied to the object tissue and the control section stops outputting the high-frequency energy when the amount of electric power exceeds a predetermined threshold value.

According to a seventh aspect of the present invention, in a high-frequency operation apparatus in the sixth aspect of the invention, the monitoring of the amount of electric power by the parameter monitoring section has the first mode of monitoring the total integrated amount of electric power in the period when the operator operates to output the high-frequency energy and the second mode of monitoring the integrated amount of electric power in the period when the high-frequency energy is actually output.

According to an eighth aspect of the present invention, in a high-frequency operation apparatus in the second aspect of the invention, the parameter monitoring section monitors the impedance at predetermined time intervals during the treatment in the first output period and the control section stops outputting the high-frequency energy and stores the impedance at the time of exceeding the first threshold value but restarts when the impedance exceeds a predetermined threshold value in the first memory but it restarts outputting high-frequency energy from the energy generating section, computes the difference value between the impedance at the restart time and the impedance stored in the first memory and controls the output of high-frequency energy according to the difference value.

According to ninth aspect of the present invention, in a high-frequency operation apparatus in the second aspect of the invention, the parameter monitoring step measures the impedance at predetermined time intervals during the treatment in the first output period, monitors if the impedance exceeds a predetermined threshold value or not and the control step stops outputting the high-frequency energy and stores the impedance at the time of exceeding the first threshold value in the first memory, and in the second output period after the first output period the control step restarts outputting the high-frequency energy, computes the difference value between the impedance at the restart time and the impedance stored in the first memory and controls the output of the high-frequency energy according to the difference value.

The invention can be more fully understood from the following detailed description when taken in conjunction with the accompanying drawings, in which:
FIG. 1 is a schematic block diagram of the first embodiment of high-frequency cauterization power supply apparatus according to the present invention, showing the configuration thereof;
FIG. 2 is a flowchart of the control operation of the control section of the first embodiment;
FIGS. 3A and 3B are schematic illustrations of the effect of the first embodiment;
FIG. 4 is a schematic block diagram of the second embodiment of high-frequency cauterization power supply apparatus according to the present invention, showing the configuration thereof;
FIG. 5 is a flowchart of the sequence of high-frequency output control of the second embodiment of the present invention;
FIG. 6 is a schematic illustration of the effect of the second embodiment;
FIG. 7 is a flowchart of the sequence of high-frequency output control of the third embodiment of the present invention;
FIG. 8 is a schematic illustration of the effect of the third embodiment; and
FIG. 9 is a schematic detailed illustration of Step S29 of FIG. 7 of the third embodiment.

Now, the present invention will be described in greater detail by referring to the accompanying drawings that illustrate preferred embodiments of the invention.

### (First Embodiment)

The first embodiment is characterized in that it monitors the change with time of the impedance as an electrical parameter that indicates the condition of the object tissue and controls the output of high-frequency energy.

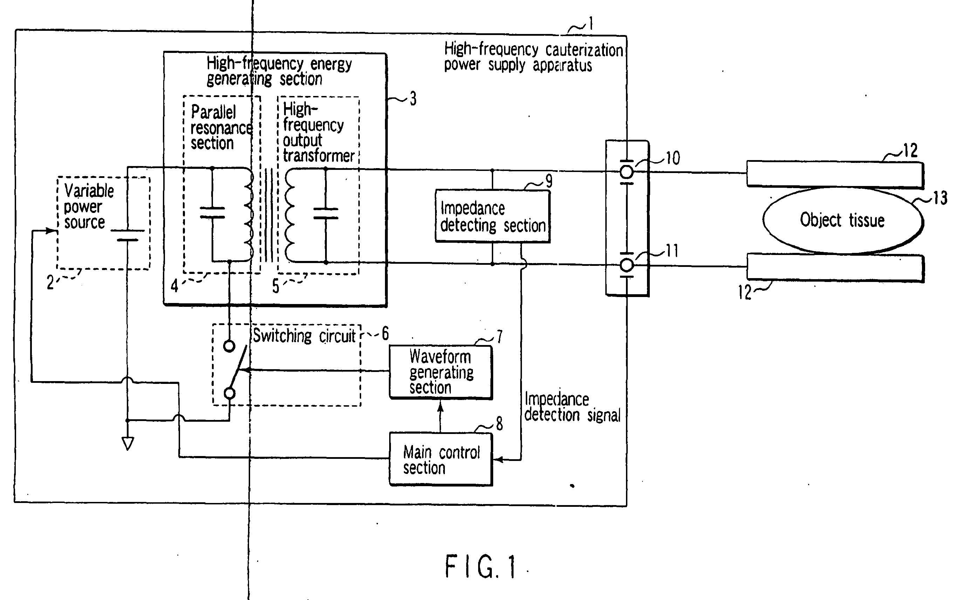

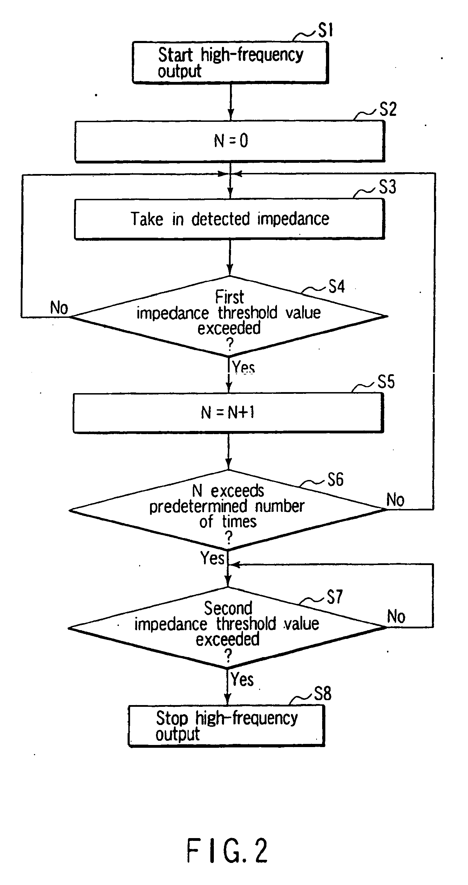

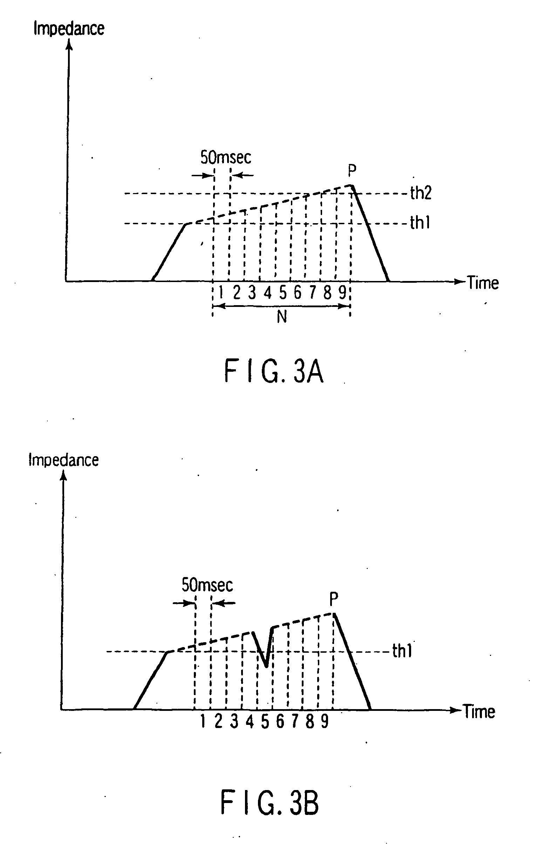

FIG. 1 is a schematic block diagram of the first embodiment of high-frequency cauterization power supply apparatus according to the present invention, showing the configuration thereof. FIG. 2 is a flowchart of the control operation of the control section 8 of the first embodiment. FIGS. 3A and 3B are schematic illustrations of the effect of the first embodiment, showing the behavior with time of the impedance as an electrical parameter that is used when sealing off a blood vessel.

Referring to FIG. 1, main control section 8 is a part that controls the operations of the component sections. A parallel resonance section 4 and a high-frequency output transformer 5 are arranged respectively at the primary side and at the secondary side of high-frequency energy generating section 3. A variable power source 2 is connected to the primary side parallel resonance section 4, which parallel resonance section 4 is adapted to remove the spurious part of the rectangular wave obtained mainly as a switching operation and boost the voltage.

The high-frequency electric current generated by the high-frequency energy generating section 3 is transmitted to electrode 12 by way of output leads 10, 11. As a result, a cauterization treatment is performed on the object tissue 13, which is the object of treatment and held between electrodes 12 with an appropriate amount of holding power. In the meantime, impedance detecting section 9 detects the impedance by dividing the detected voltage by the detected current and transmits the outcome of detection to the main control section 8 as impedance detection signal. The detected impedance changes according to the situation of cauterization of the tissue.

The main control section 8 transmits a control signal that corresponds to set conditions and the impedance detected by the impedance detecting section 9 to variable power source 2 and waveform generating section 7. The variable power source 2 outputs direct current electric power that corresponds to the control signal transmitted from the main control section 8. The waveform generating section 7 outputs a waveform (of a rectangular wave here) that corresponds to the control signal output from the main control section 8. The high-frequency energy generating section 3 generates high-frequency energy according to the operation of switching circuit 6 that is turned on and off according to the direct current electric power transmitted from the variable power source 2 and the rectangular wave transmitted from the waveform generating section 7.

When treating the object tissue, high-frequency energy is generated and output by the high-frequency energy generating section 3 and supplied to the object tissue 13 by way of the electrodes 12 under the control of the main control section 8 (Step S1 in FIG. 2). The main control section 8 substitutes 0 for the variable N that indicates the number of times exceeding a threshold value as will be described hereinafter (Step S2). Then, the main control section 8 successively takes in the impedance detected by the impedance detecting section 9 at intervals of a predetermined period (a period of 50 ms in FIG. 3A) (Step S3). The detection of the impedance is conducted successively so long as high-frequency energy is output from the high-frequency cauterization power supply apparatus 1.

The main control section 8 determines if the impedance signal it takes in exceeds the first impedance threshold value (th1) that is a specific target impedance or not (Step S4) and returns to Step S3 when the impedance signal does not exceed the threshold value but substitute 1 for the variable N when the impedance signal exceeds the threshold value (Step S5). Thereafter, the main control section 8 determines if the variable N gets to a predetermined number of times (9 times here) or not (Step S6). The main control section 8 repeats the Steps S3 through S6 until N = 9 is obtained and, when the conditional result in Step S6 is YES, it determines if the finally achieved impedance exceeds the second impedance threshold value (th2) or not (Step S7). The main control section 8 continues outputting high-frequency energy so long as the conditional result is NO but it determines that the tissue is sufficiently dried and outputs a signal for stopping output of high-frequency energy to the variable power source 2 and the waveform generating section 7 when the conditional result becomes YES (Step S8).

Note that, when the impedance level falls below the first impedance threshold value th1 while the main control section 8 is counting the number of times by which the impedance signal taken in by the main control section 8 exceeds th1 as shown in FIG. 3B (the impedance level falls below th1 at the fifth measurement in FIG. 3B), the main control section 8 determines that the behavior of the impedance is unstable due to the influence of external turbulences (and hence the dehydration of the tissue is not sufficient), resets the past count value and repeats a similar process from the beginning.

Thus, with the above-described first embodiment, if the impedance transiently changes due to some external factor or another while a cauterization treatment is being performed on the object tissue 13 with high-frequency power, it is not determined to stop the output of high-frequency power immediately at that moment and continuation/stop of the high-frequency output is determined according to the change with time of the impedance. With this arrangement, if the contact condition becomes defective at the site where the tissue is held by a forceps and the impedance transiently changes due to a generated small spark, it is possible to reliably and perfectly dehydrate the tissue to improve the reliability of sealing off a blood vessel.

### (Second Embodiment)

The second embodiment is characterized in that it monitors the change with time of the amount of electric power as an electrical parameter that indicates the condition of the object tissue and controls the output of high-frequency energy.

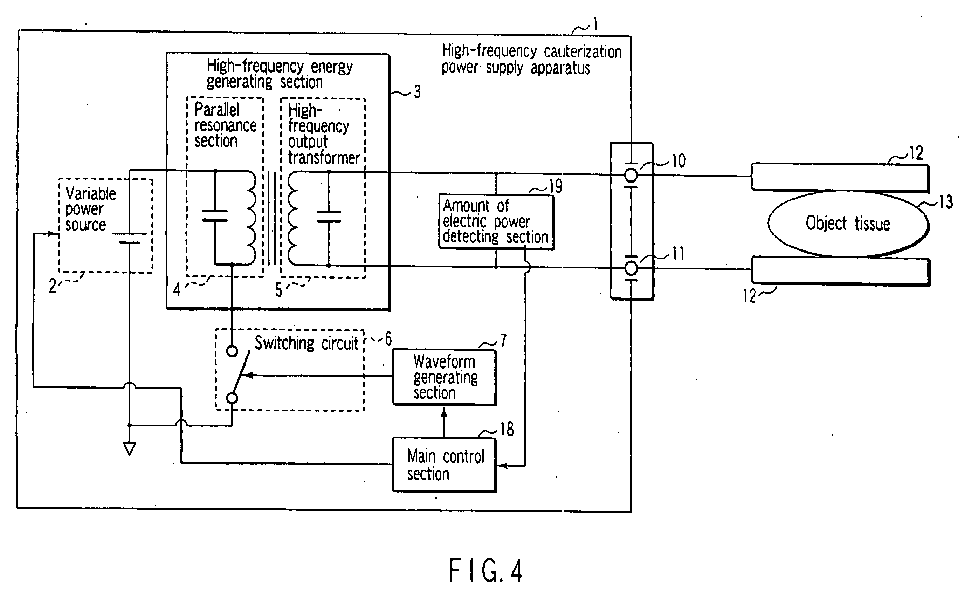

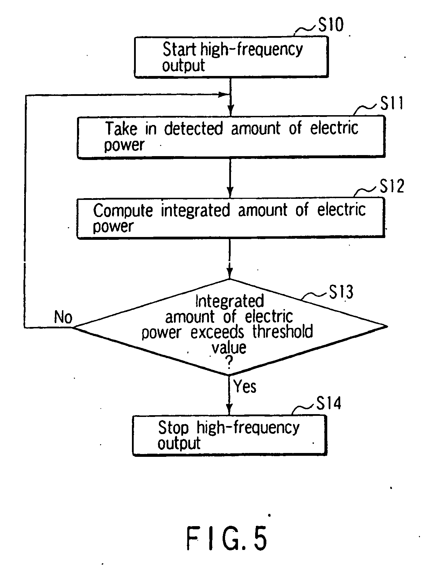

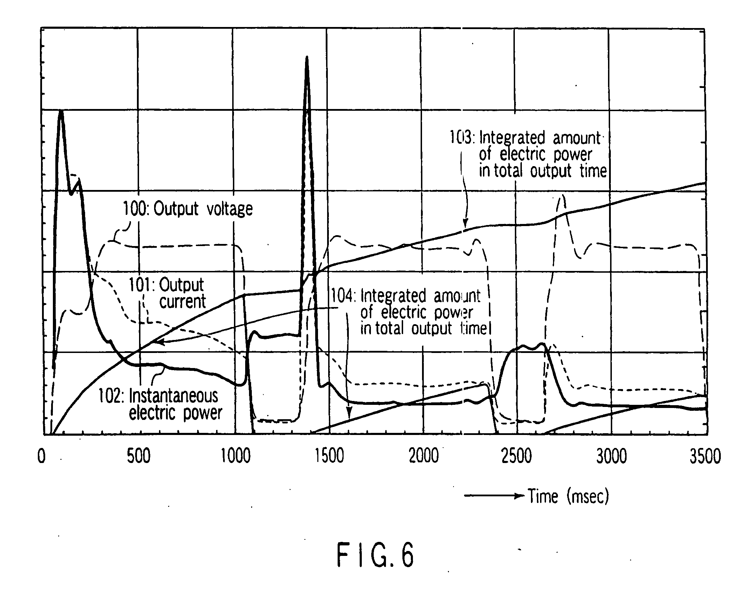

FIG. 4 is a schematic block diagram of the second embodiment of high-frequency cauterization power supply apparatus according to the present invention, showing the configuration thereof. FIG. 5 is a flowchart of the sequence of high-frequency output control of the second embodiment of the present invention. FIG. 6 is a schematic illustration of the effect of the second embodiment, showing the behavior with time of the integrated amount 103, 104 of electric power as an electrical parameter that is used when sealing off a blood vessel.

The configuration of FIG. 4 is identical with that of FIG. 1 except that the impedance detecting section 9 of FIG. 1 is replaced by an amount of electric power detecting section 19 for detecting the amount of electric power supplied to the object tissue 13. The amount of electric power detecting section 19 successively detects the output current (101 in FIG. 6) and the output voltage (100 in FIG. 6) at intervals of a predetermined period (e.g., a period of 50 ms) and detects the amount of electric power by multiplying them with each other. The amount of emitted heat at the object tissue 13 rises as the amount of electric power supplied to the object tissue 13 rises. Therefore, it is possible to monitor the condition of dehydration of the object tissue 13 by monitoring the amount of electric power.

When treating the object tissue 13, high-frequency energy is generated and output by the high-frequency energy generating section 3 and supplied to the object tissue 13 by way of the electrodes 12 under the control of the main control section 8 (Step S10).

During the treatment, the main control section 8 takes in the amount of electric power (instantaneous electric power 102 x output time) detected by the amount of electric power detecting section 19 as data. The detection of the amount of electric power is conducted successively so long as high-frequency energy is output from the high-frequency cauterization power supply apparatus 1.

Then, the main control section 8 computes the integrated amount of electric power on the basis of the amounts of electric power that are taken in (Step S12). Then, it determines if the computed integrated amount of electric power exceeds a predetermined amount of electric power threshold value (the amount of electric power as ending condition) or not (Step S13). When the conditional result is NO, the main control section 8 returns to Step S11 to repeat the subsequent steps. However, when the conditional result in Step S13 becomes YES, it determines that the tissue is sufficiently dried and outputs a signal for stopping output of high-frequency energy to the variable power source 2 and the waveform control section 7 (Step S14).

FIG. 6 shows that the amount of electric power is monitored in two different modes. The first one is a mode in which the total integrated amount of electric power 103 is monitored for the total output time (the period during which the operator continuously operates for outputting high-frequency energy) and the second one is a mode in which the integrated amount of electric power 104 is monitored for the total period during which high-frequency energy is actually output (high-frequency energy is output intermittently as on and off are repeated). While the threshold value set for determining stop or continuation of the high-frequency energy output is high in the first mode from the viewpoint of elapsed time as the amount of electric power is obtained by multiplication, the threshold value is low in the second mode from the viewpoint elapsed time. However, the obtained effect does not show any difference between the two modes.

A large amount of electric power is required in the initial stages of high-frequency energy output because the object tissue contains water to a large extent. However, as the water is evaporated and dispersed to become sufficiently dry, the impedance of the tissue rises so that practically no electric current flows. For this reason, a relatively high threshold value is set for the amount of electric power for shifting to an inactive period in the initial stages of output and a relatively low threshold value is set for the amount of electric power as the output gradually progresses.

Thus, with the above-described second embodiment, the condition of the object tissue 13 is determined according to the amount of electric power supplied to the object tissue 13 so that it is possible to reduce the variances in the level of sealing off the blood vessel due to the nature and the condition of the object tissue 13 and the influence of external factors can be minimized. In other words, it is possible to realize stable performances for sealing off a blood vessel regardless of the condition of the object tissue 13.

### (Third Embodiment)

The third embodiment is characterized in that it monitors the difference of two impedances detected at different timings as an electrical parameter that indicates the condition of the object tissue and controls the output of high-frequency energy. More specifically, the output of high-frequency energy is controlled according to the difference value D between the impedance Z1 at the first time point (at the N-th time point) of stopping the output of high-frequency energy and the impedance Z2 at the next time point (at the N+1-t time point) of starting the output of high-frequency energy. The list shown below describes the electrical parameter in detail.
A: control of the output of high-frequency energy according to the level of impedance Z1
   (1) When Z1 is lower than a specific impedance threshold value, the cauterization of the part held by the forceps is insufficient. In this case, the treatment control parameter is so controlled as to raise the amount of electric power in the next step regardless of the information on the difference value D.
   (2) When Z1 is higher than the specific impedance threshold value, the dehydration of the part held by the forceps has bee practically completed. However, the cauterization may not be sufficient in the peripheral tissue. Then, the treatment control parameter (the impedance threshold value) is determined in the next step by taking the information on the difference value D into consideration. B: determination of the condition according to the difference value D and the Z1/Z2 relationship (1) When D is greater than the threshold value and Z1 < Z2, the part held by the forceps and the peripheral tissue have been dehydrated sufficiently. Therefore, it is not necessary to continue the output of high-frequency energy and hence the output is made inactive or stopped.
   (2) When D is greater than the threshold value and Z1 > Z2, the cauterization of the part held by the forceps has been completed to a certain extent but the dehydration of the peripheral tissue is insufficient or the cauterization of the part held by the forceps is insufficient. Therefore, it is necessary to continue the output of high-frequency energy and hence the output is made inactive or stopped.
   (3) When D is smaller than the threshold value and Z1 < Z2, the part held by the forceps and that of the peripheral tissue are sufficiently dehydrated. Therefore, it is not necessary to continue the output of high-frequency energy and hence the output is made inactive or stopped.
   (4) When D is smaller than the threshold value and Z1 > Z2, the part held by the forceps and that of the peripheral tissue are dehydrated substantially to a sufficient level but the cauterization needs to be continued a little. Therefore, it is so controlled that the output of high-frequency energy is continued but the amount of electric power to be supplied to the tissue is reduced.

As described above, with the third embodiment, the condition of the tissue is determined on the basis of the combinations of the three factors of the impedance Z1 at the first time point of stopping the output of high-frequency energy, the impedance Z2 at the next time point of starting the output of high-frequency energy and the difference thereof. Therefore, it is possible to determine the condition of the tissue in a more detailed manner and elaborately control the output according to the condition of the tissue. In other words, it is possible to keep sealing performances to a same level (reduce variances) regardless of the condition of the object tissue.

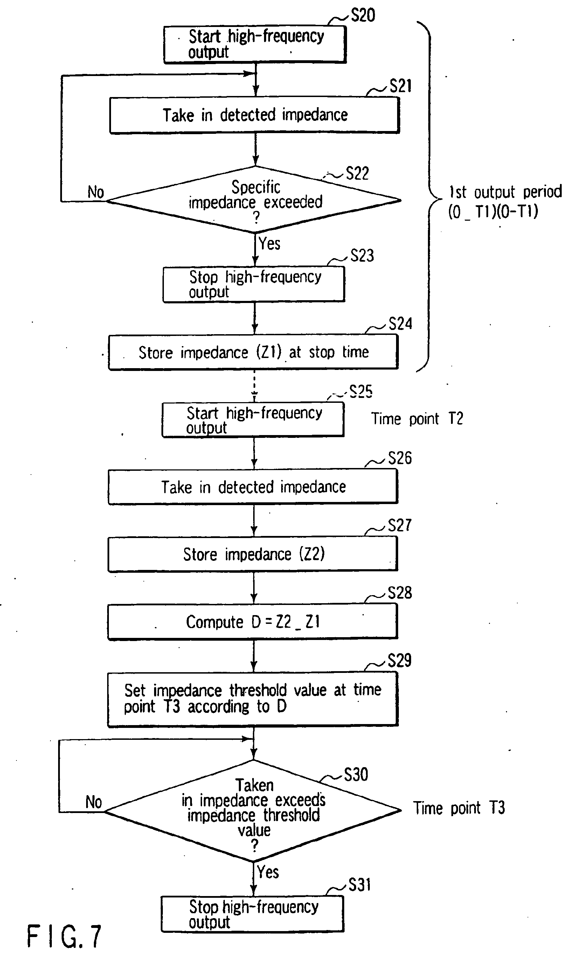

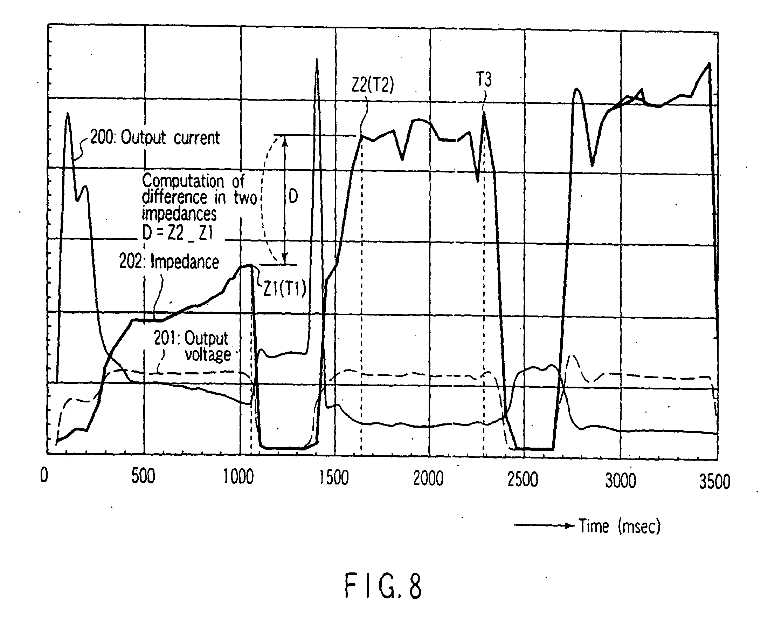

Now, a situation where the technique of the third embodiment is applied to an actual output control will be described below. FIG. 7 is a flowchart of the sequence of high-frequency output control of the third embodiment of the present invention. FIG. 8 is a schematic illustration of the effect of the third embodiment, showing the behavior of the electrical parameter, which is the impedance 202 to be more accurate, in the course of time when a blood vessel is sealed off in a surgical operation.

The third embodiment has a configuration basically same as the first embodiment (FIG. 1). Particularly, it is same as the first embodiment in that the main control section 8 takes in the signal detected by the impedance detecting section 9 and ultimately determines continuation or stop of the output of high-frequency energy but different from the first embodiment in terms of the technique it uses.

When treating the object tissue 13, high-frequency energy is generated by and output from the high-frequency energy generating section 3 under the control of the main control section 8 and supplied to the object tissue 13 by way of the electrodes 12 (Step S20).

The main control section 8 takes in the impedance detected by the impedance detecting section 9 (Step S21) and determines if it exceeds the specific impedance threshold value or not (Step S22). When the detected impedance does not exceed the threshold value, it returns to Step S21, and when the detected impedance exceeds the threshold value, it outputs a signal for stopping the high-frequency energy output to the variable power source 2 and the waveform control section 7 (Step S23). At this time, the absolute value of the impedance (Z1) observed at the time point when the output of high-frequency energy is stopped is stored in the memory in the inside of the apparatus (Step S24).

In this way, it is determined if the output of high-frequency electric power in the first output period (0 to T1) from the start is to be stopped or continued by comparing the impedance that is taken in with the specific impedance threshold value and the output of high-frequency energy is stopped at the time point when the impedance that is taken in exceeds the specific impedance threshold value. However, the condition for stopping the output may alternatively be a predetermined time.

Thereafter, after a predetermined time period (T1 to T2) of inactivating the output of high-frequency energy, the high-frequency cauterization power supply apparatus 1 again generates and outputs high-frequency electric power (Step S25). At this time, the impedance (Z2) of the time point (T2) of starting the output of high-frequency energy is detected by the impedance detecting section 9 and taken in by the main control section 8 (Step S26) and the absolute value of the observed impedance is stored in the memory in the inside of the apparatus (Step S27). The main control section 8 reads out the impedances Z1 and Z2 stored in the memory and computes the difference value of the two impedances D = Z2 - Z1 (Step S28). Then, the main control section 8 sets the impedance threshold value for determining if the output of high-frequency energy is to be intercepted or continued (the determining threshold value at time point T3) according to the computed value of D (Step S29). Then, it compares the impedance value that is taken in from time to time and the impedance threshold value set in Step S29 (Step S30). If the impedance signal that is dependent on the difference value D exceeds the set threshold value at T3, it outputs a signal for stopping the output of high-frequency energy to the variable power source 2 and the waveform control section 7 (Step S31). Thereafter, the above-described process is continued until a situation where the tissue is ultimately and completely dehydrated is detected.

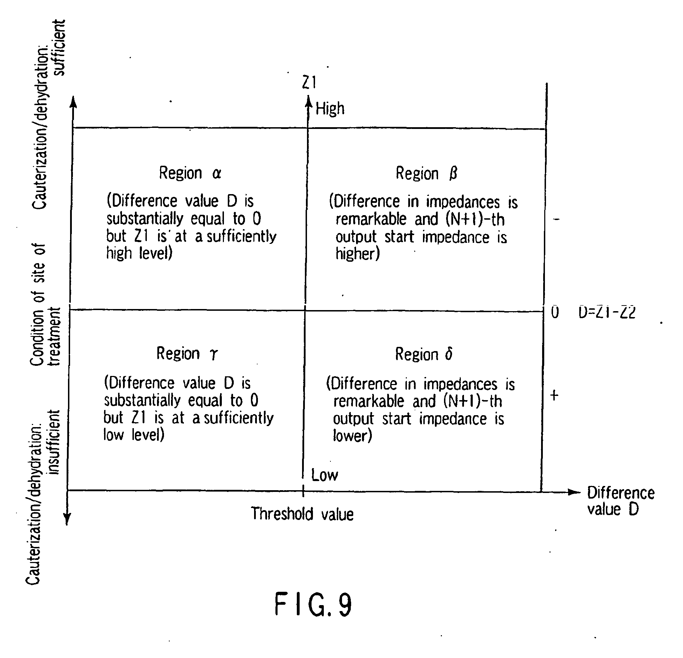

FIG. 9 is a schematic detailed illustration of Step S29 of FIG. 7. As described above, in the third embodiment controls the output of high-frequency energy according to the three factors of the impedance Z1 when the output of high-frequency energy is stopped at the N-th time point and the impedance Z2 when the output of high-frequency energy is started at the N+1-th time and the difference value D of Z1 and Z2. A step of determining if the difference value D falls below a predetermined threshold value (and hence there is practically no difference between D1 and D2) may be added. Then, when the difference value D falls below a predetermined threshold value (the value of D is found in region α or region γ in FIG. 9), the impedance threshold value is set by considering the absolute value of Z1 to control the output of high-frequency energy.

FIG. 9 summarily illustrates the above description. In FIG. 9, the difference value D is substantially equal to 0 but Z1 is at a sufficiently high level in the region α. In this case, the output of high-frequency energy is controlled and lowered or stopped. The difference value D between Z1 and Z2 is remarkably large and the impedance Z2 at the (N+1)-th time point when the output of high-frequency energy is started is higher than the impedance Z1 at the N-th time point when the output of high-frequency energy is stopped. In this case, the output of high-frequency energy is controlled and lowered or stopped. The difference value D is substantially equal to 0 but Z1 is at a sufficiently low level in the region γ. In this case, the output of high-frequency energy is controlled and raised or continued for a longer time. The difference value D between Z1 and Z2 is remarkably large and the impedance Z2 at the (N+1)-th time point when the output of high-frequency energy is started is lower than the impedance Z1 at the N-th time point when the output of high-frequency energy is stopped. In this case, the output of high-frequency energy is controlled and raised or continued for a longer time.

The third embodiment is adapted to regulate the impedance threshold value for determining interception or continuation of the output of high-frequency energy according to the impedance difference value D. However, a similar effect can be obtained if the factor to be used for the regulation is replaced by the electric power value, the electric current value and the electric voltage value at the N-th output.

Particularly, when a large tissue is to be held and treated, the impedance is low in the initial stages. Additionally, the current density (the power density) of the site of treatment falls so that the cauterization does not progress remarkably and the output of high-frequency energy needs to be continued to prolong the treatment time. Although not described in detail, the longest permissible output time may be separately defined for each output period and this embodiment may be combined with such an arrangement to provide a particularly remarkable advantage.

## Claims

1. A high-frequency operation apparatus, comprising:
a high-frequency energy generating section (3) which generates and outputs high-frequency energy to be used for treating an object tissue (13);
a parameter monitoring section (9) which monitors the change with time of an electrical parameter indicting the condition of the object tissue (13) when the high-frequency energy output from the high-frequency energy generating section (3) is supplied to the object tissue (13); and
a control section (8) which controls the output of the high-frequency energy according to the outcome of the monitoring by the parameter monitoring section (9),
wherein the parameter monitoring section (9) measures the impedance at predetermined time intervals during the treatment, monitors if the impedance exceeds a first threshold value or not, wherein
when the number of times of exceeding the first threshold value gets to a predetermined number of times, the control section (8) stops the output of the high-frequency energy, **characterized in that**
the parameter monitoring section (9) resets a count value obtained by the past measurement but continues the measurement of the impedance when the impedance falls below the first threshold value during the measurement of the impedance.

2. The apparatus according to claim 1, **characterized in that**
the parameter monitoring section (9) monitors the change with time of the impedance that indicates the condition of the object tissue (13).

3. The apparatus according to claim 1, **characterized in that**
the parameter monitoring section (9) determines if the impedance exceeds a second threshold value that is different from the first threshold value or not after the number of times by which the impedance exceeds the first threshold value gets to a predetermined number of times and the control section (8) stops outputting the high-frequency energy when the impedance exceeds the second threshold value.

4. The apparatus according to claim 1, **characterized in that**
the parameter monitoring section (9) monitors the amount of electric power of the high-frequency energy being applied to the object tissue (13) and the control section (8) stops outputting the high-frequency energy when the amount of electric power exceeds a predetermined threshold value.

5. The apparatus according to claim 4, **characterized in that**
the monitoring of the amount of electric power by the parameter monitoring section (9) has a first mode of monitoring the total integrated amount of electric power in the period when the operator operates to output the high-frequency energy and a second mode of monitoring the integrated amount of electric power in the period when the high-frequency energy is actually output.

6. The apparatus according to claim 2, **characterized in that**
the parameter monitoring section (9) measures the impedance at predetermined time intervals during the treatment in a first output period and monitors if the impedance exceeds a predetermined threshold value or not, and the control section (8) stops outputting the high-frequency energy and stores the impedance at the time of exceeding the first threshold value in the first memory, and in a second output period after the first output period the control section restarts outputting high-frequency energy from the energy generating section (3), computes the difference value between the impedance at the restart time and the impedance stored in the first memory and controls the output of the high-frequency energy according to the difference value.

7. The apparatus according to claim 6, **characterized in that**
after the control section (8) stops the first output, the control section (8) restarts the second output in a predetermined time.

## Patentansprüche

1. Hochfrequenzbetätigungsvorrichtung aufweisend:
einen Hochfrequenzenergieerzeugungsabschnitt (3), der Hochfrequenzenergie zur Verwendung zum Behandeln eines Zielgewebes (13) erzeugt und ausgibt;
einen Parameterüberwachungsabschnitt (9), der die Änderung über der Zeit eines den Zustand des Zielgewebes (13) anzeigenden elektrischen Parameters überwacht,
wenn die Hochfrequenzenergieausgabe des Hochfrequenzenergieerzeugungsabschnitts (3) dem Zielgewebe (13) zugeführt wird; und
einen Steuerungsabschnitt (8), der die Ausgabe der Hochfrequenzenergie gemäß dem Ergebnis der Überwachung durch den Parameterüberwachungsabschnitt (9) steuert,
wobei der Parameterüberwachungsabschnitt (9) die Impedanz in vorbestimmten Zeitintervallen während der Behandlung misst, überwacht, ob die Impedanz einen ersten Schwellwert übersteigt oder nicht, wobei
wenn die Anzahl der Übersteigungen des ersten Schwellwerts eine vorbestimmte Anzahl erreicht, der Steuerungsabschnitt (8) die Ausgabe der Hochfrequenzenergie beendet, **dadurch gekennzeichnet, dass**
der Parameterüberwachungsabschnitt (9) einen bei der vergangenen Messung erlangten Zählwert zurücksetzt, aber die Messung der Impedanz fortsetzt, wenn die Impedanz während der Messung der Impedanz unter den ersten Schwellwert fällt.

2. Vorrichtung nach Anspruch 1, **dadurch gekennzeichnet, dass** der Parameterüberwachungsabschnitt (9) die Änderung über der Zeit der Impedanz, die den Zustand des Zielgewebes (13) anzeigt, überwacht.

3. Vorrichtung nach Anspruch 1, **dadurch gekennzeichnet, dass** der Parameterüberwachungsabschnitt (9) bestimmt, ob die Impedanz einen zweiten Schwellwert, der sich von dem ersten Schwellwert unterscheidet, übersteigt oder nicht nachdem die Anzahl der Impedanzübersteigungen des ersten Schwellwerts eine vorbestimmte Anzahl erreicht und der Steuerungsabschnitt (8) die Ausgabe der Hochfrequenzenergie beendet, wenn die Impedanz den zweiten Schwellwert übersteigt.

4. Vorrichtung nach Anspruch 1, **dadurch gekennzeichnet, dass** der Parameterüberwachungsabschnitt (9) den Betrag der an das Zielgewebe (13) applizierten elektrischen Leistung überwacht und der Steuerungsabschnitt (8) die Ausgabe der Hochfrequenzenergie beendet, wenn der Betrag der elektrischen Leistung einen vorbestimmten Schwellwert übersteigt.

5. Vorrichtung nach Anspruch 4, **dadurch gekennzeichnet, dass** die Überwachung des Betrags der elektrischen Leistung durch den Parameterüberwachungsabschnitt (9) aufweist einen ersten Modus eines Überwachens des gesamten integrierten Betrags von elektrischer Leistung in dem Zeitraum während dem die Bedienperson die Ausgabe der Hochfrequenzenergie veranlasst und einen zweiten Modus eines Überwachens des integrierten Betrags elektrischer Leistung in dem Zeitraum während dem die Hochfrequenzenergie tatsächlich ausgegeben wird.

6. Vorrichtung nach Anspruch 2, **dadurch gekennzeichnet, dass** der Parameterüberwachungsabschnitt (9) die Impedanz in vorbestimmten Zeitintervallen während der Behandlung in einem ersten Ausgabezeitraum misst und überwacht, ob die Impedanz einen vorbestimmten Schwellwert übersteigt oder nicht, und der Steuerungsabschnitt (8) die Ausgabe der Hochfrequenzenergie beendet und die Impedanz zum Zeitpunkt des Überschreitens des ersten Schwellwerts in dem ersten Speicher speichert, wobei der Steuerungsabschnitt in einem zweiten Ausgabezeitraum nach dem ersten Ausgabezeitraum die Ausgabe von Hochfrequenzenergie durch den Energieerzeugungsabschnitt (3) wieder beginnt, den Differenzwert zwischen der Impedanz zu dem Wiederbeginnzeitpunkt und der in dem ersten Speicher gespeicherten Impedanz berechnet und die Ausgabe der Hochfrequenzenergie gemäß dem Differenzwert steuert.

7. Vorrichtung nach Anspruch 6, **dadurch gekennzeichnet, dass** nachdem der Steuerungsabschnitt (8) die erste Ausgabe beendet, der Steuerungsabschnitt (8) in einer vorbestimmten Zeit die zweite Ausgabe wieder startet.

## Revendications

1. Appareil opérant à haute fréquence, comprenant :
une section de génération d'énergie haute fréquence (3) qui génère et délivre en sortie une énergie haute fréquence destinée à être utilisée pour traiter un tissu objet (13) ;
une section de surveillance de paramètre (9) qui surveille le changement avec le temps d'un paramètre électrique indiquant l'état du tissu objet (13) lorsque l'énergie haute fréquence délivrée en sortie de la section de génération d'énergie haute fréquence (3) est amenée jusqu'au tissu objet (13) ; et
une section de commande (8) qui commande la délivrance en sortie de l'énergie haute fréquence en fonction du résultat de la surveillance par la section de surveillance de paramètre (9), dans lequel la section de surveillance de paramètre (9) mesure l'impédance à intervalles temporels prédéterminés pendant le traitement, surveille si l'impédance dépasse une première valeur seuil ou non, comment,
lorsque le nombre de fois de dépassement de la première valeur seuil atteint un nombre de fois prédéterminé, la section de commande (8) stoppe la délivrance en sortie de l'énergie haute fréquence, **caractérisé en ce que**
la section de surveillance de paramètre (9) réinitialise une valeur de comptage obtenue par la mesure passée mais continue la mesure de l'impédance lorsque l'impédance chute en-dessous de la première valeur seuil pendant la mesure de l'impédance.

2. Appareil selon la revendication 1, **caractérisé en ce que**
la section de surveillance de paramètre (9) surveille le changement avec le temps de l'impédance qui indique l'état du tissu objet (13).

3. Appareil selon la revendication 1, **caractérisé en ce que**
la section de surveillance de paramètre (9) détermine si l'impédance dépasse une deuxième valeur seuil qui est différente de la première valeur seuil ou non après que le nombre de fois où l'impédance dépasse la première valeur seuil atteint un nombre de fois prédéterminé et la section de commande (8) stoppe la délivrance en sortie de l'énergie haute fréquence lorsque l'impédance dépasse la deuxième valeur seuil.

4. Appareil selon la revendication 1, **caractérisé en ce que**
la section de surveillance de paramètre (9) surveille la quantité d'énergie électrique de l'énergie haute fréquence étant appliquée au tissu objet (13) et la section de commande (8) stoppe la délivrance en sortie de l'énergie haute fréquence lorsque la quantité d'énergie électrique dépasse une valeur seuil prédéterminée.

5. Appareil selon la revendication 4, **caractérisé en ce que**
la surveillance de la quantité d'énergie électrique par la section de surveillance de paramètre (9) a un premier mode de surveillance de la quantité totale intégrée d'énergie électrique sur la période pendant laquelle l'opérateur opère pour délivrer en sortie l'énergie haute fréquence et un deuxième mode de surveillance de la quantité intégrée d'énergie électrique sur la période pendant laquelle l'énergie haute fréquence est effectivement délivrée en sortie.

6. Appareil selon la revendication 2, **caractérisé en ce que**
la section de surveillance de paramètre (9) mesure l'impédance à intervalles temporels prédéterminés pendant le traitement sur une première période de délivrance en sortie et surveille si l'impédance dépasse une valeur seuil prédéterminée ou non, et la section de commande (8) stoppe la délivrance en sortie de l'énergie haute fréquence et mémorise l'impédance au moment du dépassement de la première valeur seuil dans la première mémoire, et sur une deuxième période de délivrance en sortie après la première période de délivrance en sortie, la section de commande redémarre la délivrance en sortie de l'énergie haute fréquence provenant de la section de génération d'énergie (3), calcule la valeur de différence entre l'impédance au moment du redémarrage et l'impédance mémorisée dans la première mémoire et commande la délivrance en sortie de l'énergie haute fréquence en fonction de la valeur de différence.

7. Appareil selon la revendication 6, **caractérisé en ce que**
après que la section de commande (8) a stoppé la première délivrance en sortie, la section de commande (8) redémarre la deuxième délivrance en sortie dans un délai prédéterminé.
